# EUROPEAN PATENT SPECIFICATION

(11) **EP 0 574 961 B1**
(45) Date of publication and mention of the grant of the patent: **08.05.1996**
(21) Application number: 93113064.5
(22) Date of filing: 13.07.1990
(51) Int. Cl.: A61M 25/02

(54) **Catheter holder**
Katheterhalterung
Support pour cathéter

(30) Priority: 14.07.1989 GB 8916191; 09.08.1989 GB 8918171
(43) Date of publication of application: 22.12.1993
(62) Divisional of application: 90307707.1
(73) Proprietor: E.R. SQUIBB & SONS, INC., Princeton New Jersey 08543-4000 (US)
(72) Inventor: Plass, Ronald Andrew, Sussex (GB); Hollands, Keith G.M., Sussex (GB)
(74) Representative: Cook, Anthony John

(56) References cited:
- EP-A- 0 122 344
- GB-A- 2 211 417
- US-A- 3 924 636
- US-A- 4 074 397
- US-A- 4 333 468

## Description

As a result of various medical procedures, catheters often are employed to drain body fluids or administer substances. Various devices have been developed for the purpose of securing a catheter or other tubing to the body of the patient to prevent accidental dislodgement. Devices which rely on an adhesive attachment to the catheter or tubing are shown, for example, by Mellor in U.S. Patent 3,826,254, Haerr in U.S. Patent 4,122,857, Geist in U.S Patent 4,333,468, Brown in U.S. Patent 4,484,914, Moseley in U.S Patent 4,460,356, Vaillancourt in U.S. Patents 4,801,296 and 4,830,914, Hesketh in U.K. Patent Application 2,211,417, and Muller in European Patent Application 206,558. Other devices have been developed which secure the catheter by a means of interengageable fabric such as Velcro as shown, for example, by Boyd in U.S. Patent 3,834,380, Rosin in U.S. Patent 4,074,397, Kaplan et al. in U.S. Patent 4,096,863, Womack in U.S. Patent 4,416,664, Hubbard et al. in U.S. Patents 4,571,245 and 4,617,017 and Campbell in U.S. Patent 4,799,923. Other devices have been developed which rely on a mechanical means to secure the catheter as shown, for example, by Edwards in U.S. Patent 4,360,025, Brown in U.S. Patent 4,378,012, Beran in U.S. Patent 4,392,857, Gordon in U.S. Patent 4,397,647, Taheri in U.S. Patent 4,586,919, Weeks in U.S. Patent 4,645,492, Nowak et al. in U.S. Patent 4,699,616, Bierman in U.S.Patent 4,711,636, Cameron et al. in U.S. Patent 4,717,385, and Hesketh in U.S. Patent 4,874,380.

It has been proposed in GB 2,211,417A, which is considered to represent the closest prior art with respect to the present invention that a catheter retaining device should include a pad having a hole therein, one surface of the pad being adhesive for adhering to the body of a patient, and the other surface of the pad mounting a tab element adjacent said hole. The element is foldable relative to the pad, and the surface of the tab remote from the pad is adhesive. The arrangement is such that the adhesive tab can he folded around and adhere to a catheter which extends through the hole, so retaining the catheter in position.

According to the invention, there is provided a catheter holder which comprises a pad of medical grade adhesive material having one surface for attachment to the skin of a wearer, tape means secured to the other surface of the pad and providing a pair of tape sections, which extend from said other surface and are aligned with each other, there being adhesive on a surface of at least one tape section, characterised in that (a) a hole in the pad between the tape sections is provided, through which the catheter may be passed in a direction transversely of the pad, and (b) a surface of one tape section has multiple use adhesive thereon and faces towards the other tape section such that the facing surfaces of the tape sections can be temporarily adhered to each other on each side of the catheter to thereby encase the catheter and hold it in such a position that it extends transversely of the pad, the distal ends of the tape sections being free of said adhesive so that they can he used to facilitate peeling the tape sections apart and said adhesive also being arranged to adhesively engage the catheter to prevent longitudinal movement thereof relative to the pad

Optionally, the pad may have an opening or hole near the point of attachment of the tapes, so that the catheter can be threaded through the pad. Alternatively, the pad may be slit inwardly from its edge with the slit joining a hole. This arrangement enables a catheter to be engaged with the device without a threading operation. It is often necessary to avoid threading, e.g. where one end of the catheter is attached internally of the wearer and the other end is attached to a container for the discharge of exudate.

An important advantage of a preferred embodiment of this invention is that the catheter can be satisfactorily held and re-held after adjustment whether it extends from the body of the wearer substantially perpendicular to the skin, or at some lesser angle.

The invention will be better understood from the following non-limiting description of an example thereof given with reference to the accompanying drawings in which:-
Figure 1 is a view from above of one example of a catheter holder in accordance with the invention having a circular pad of medical grade adhesive, and showing tapes thereof separated;
Figure 2 is similar to Figure 1 but shows a catheter extending through the pad and the tapes in their unjoined inoperative position, and
Figure 3 is similar to Figures 1 and 2 but shows the two tapes stuck together to surround and grip the catheter and hold it transverse to the medical grade adhesive pad.

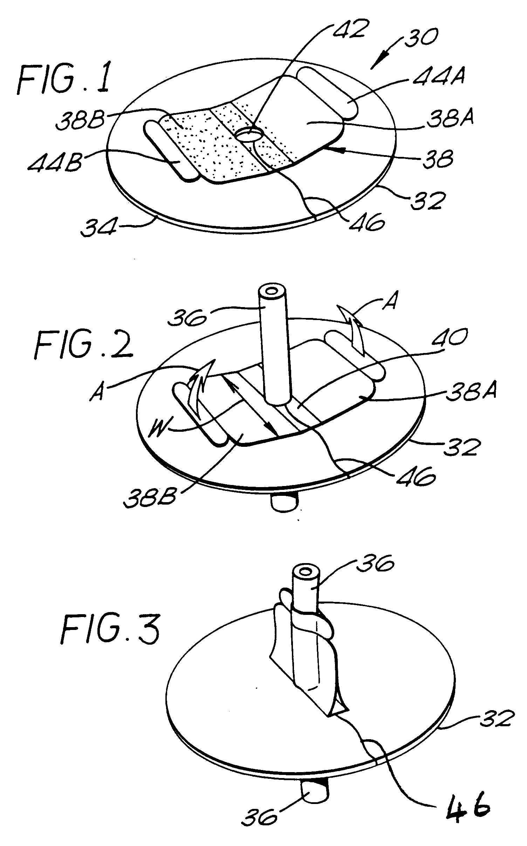

The catheter holder 30 illustrated in Figures 1-3 includes a pad 32 of medical grade adhesive. This is shown as circular but could be oval or of any other suitable shape. The pad 32 may be made of the materials as are mentioned in the succeeding paragraph. A film 34 of synthetic plastics material overlaps one surface of the pad 32. The other surface is intended for direct application to the skin of a wearer, surrounding the exit point from the body of the catheter 36. A pair of tape or strip members 38A, 38B are attached to the film 34. The tapes are preferably synthetic plastics strips. They may be formed by a single strip 38 joined to the film 34 over an intermediate region 40. The join may be by adhesive or plastics heat welding or plastics RF welding. The pad 32 has a central hole 42 and this is connected to the periphery of the pad by a curved slit 46. The slit 46 extends completely through the thickness of the pad 32, the film 34 and the tape 38. It enables a catheter to be passed through the hole 42 without either end of the catheter having to be freed or disconnected. This is an essential requirement in some circumstances where a catheter is used.

Any of the known medical grade pressure sensitive adhesives can be employed as the pad 32. Preferably, adhesive material 12 is formulated by blending one or more water soluble or swellable hydrocolloids with a polyisobutylene or a mixture of polyisobutylenes or a mixture of one or more polyisobutylenes and one or more non-acrylic elastomers. Other materials can be included within the adhesive formulations such as mineral oil, tackifiers, antioxidants, cohesive strengthening agents, and pharmaceutically active materials such as anti-inflammatory agents, antiseptics, or materials having skin healing or soothing properties. Suitable adhesive formulations are taught by Chen in U.S. Patent 3,339,546, Chen et al in U.S. Patent 4,192,785, Pawelchak et al in U.S. Patent 4,393,080, Doyle et al in U.S. Patent 4,551,490 and by Keyes et al in U.S. Patent 4,762,738. As disclosed in these references, suitable water soluble and water swellable hydrocolloids include sodium carboxymethylcellulose, pectin, gelatin, guar gum, locust bean gum, gum karaya, and mixtures thereof. Suitable cohesive strengthening agents include water-insoluble cross-linked sodium carboxy-methylcellulose, water-insoluble cross-linked dextran, etc. Suitable non-acrylic elastomers include butyl rubber and styrene radial or block copolymers such as styrene-butadiene-styrene (S-B-S) and styrene-isoprene-styrene (S-I-S) block type copolymers.

Multi-use refers to the ability of the adhesive to retain its tack after it is peeled from a surface so that it can be refastened. Such adhesives are commercially available, for example from the 3M Company, and are employed in various products such as adhesive tabs on diapers.

An area of the tape 38 (the area being indicated by dots in Figure 1) is coated with a multi-use adhesive. At each end of the tape is a tab 44A, 44B, which may be coloured differently from the remainder of the tape 38 or may be of a different material. Alternatively, the tab may be formed by attaching a facing to the tape 38 over the areas 44A and 44B. The tabs are non-adhesive and in use are gripped to peel apart the adhered tapes 38A, 38B. As will be seen, the bulk of the area of the tape 38A is free of adhesive; this allows the tapes 38A and 38B to be peeled apart.

In use, as indicated by the arrows A in Figure 2, the two tapes 38A and 38B can be folded up to encase a catheter 36 which extends out of the wearer's body substantially perpendicularly to the plane of the pad 32, so holding the catheter in the desired position (Figure 3).

Although not shown in the Figures, the adhesive on the tapes 38A, 38B is normally covered with a strippable protective release paper or film, to avoid the adhesive picking up dirt or being degraded prior to use.

In one preferred embodiment of the catheter holder illustrated in Figures 1-3, the pad has a diameter of about 80 to 100 mm, a thickness of about 0.8 to 2.0 mm, and the tapes are about 28 to 45 mm wide. The hole 42 may be about 7.5 mm in diameter, or such other value as may accommodate the required catheter, and the length of the tape 38 measured flat may be about 60 to 70 mm. Of this length, the tabs 44A, 44B, each occupy about 6 to 8 mm. These dimensions have been found satisfactory for a variety of applications, but of course the invention can be carried into effect in catheter holders which are either larger or smaller than these dimensions. In general, it is desirable that the width W (Figure 2) of the tapes should be about 4d to 6.5d, preferably 5d to 6d, where d is the diameter of the hole 42.

The catheter holder shown in the Figures may be integrated with or securely connected to an ostomy coupling element constructed for co-operation with a complementary coupling element on a bile bag. The pad and bag are arranged so that the free end of the catheter empties into the bag. The resulting combination can be termed a "bile kit" and provides a means whereby bile may be drained from a patient into a bag, and the bag may be removed, emptied, and replaced with a fresh bag, all without disturbing the catheter and without detaching the pad 32 from the wearer.

It will be understood that modifications may be made to the catheter holders particularly described and illustrated herein, without departing from the invention, and within the meaning of EPC Article 69 and its Protocol. For example, the tape members need not be of synthetics plastics material but could be of other material which could carry a suitable multi-use adhesive. The shape and positioning of the tape members on the pad 32 need not be precisely as illustrated. In certain circumstances it may be practical to dispense with the cover film 34. Gripping tabs other than those of the kind illustrated in the Figures may be utilized. Also, the holders are useful for securing other types of tubing, wires, etc., in addition to drainage catheters, to the patient as may be required.

## Claims

1. A catheter holder which comprises a pad (32) of medical grade adhesive material having one surface for attachment to the skin of a wearer, tape means secured to the other surface of the pad and providing a pair of tape sections (38A,38B), which extend from said other surface and are aligned with each other, there being adhesive on a surface of at least one tape section (38A or 38B), characterised in that (a) a hole (42) in the pad between the tape sections is provided, through which the catheter (36) may be passed in a direction transversely of the pad (32), and (b) a surface (38A or 38B) of one tape section has multiple use adhesive thereon and faces towards the other tape section (38B or 38A) such that the facing surfaces of the tape sections can be temporarily adhered to each other on each side of the catheter to thereby encase the catheter and hold it in such a position that it extends transversely of the pad, the distal ends (44A, 44B) of the tape sections being free of said adhesive so that they can be used to facilitate peeling the tape sections apart and said adhesive also being arranged to adhesively engage the catheter to prevent longitudinal movement thereof relative to the pad (32).

2. A catheter holder according to claim 1 in which the pad is slit (46) inwardly from its edge with the slit joining the hole.

3. A catheter holder according to claim 1 or 2 in which the medical grade adhesive material (32) is a plastics adhesive material comprising a blend of a water soluble or water swellable hydrocolloid and a water insoluble viscous elastic binder.

4. A catheter holder according to any preceding claim in which the width of either of the tape sections (38A, 38B) is from 4 to 6.5d where d is the diameter of the hole (42).

5. A catheter holder according to claim 4 in which the width of the tape sections (38A, 38B) is from 5d to 6d.

6. A catheter holder according to any preceding claim in combination with a catheter and a bag for receiving bile conducted into the bag via the catheter, the bag being designed to be held onto the wearer by the medical grade adhesive pad (32).

## Patentansprüche

1. Katheterhalterung mit einem Polster (32) aus medizinischem Haftmaterial mit einer Oberfläche zur Befestigung auf der Haut des Trägers, einer Bandeinrichtung, die an der anderen Oberfläche des Polsters befestigt ist und ein Paar Bandabschnitte (38A, 38B) hat, die von der anderen Oberfläche verlaufen und zueinander ausgerichtet sind, wobei sich Haftmittel auf einer Oberfläche mindestens eines Bandäbschnitts (38A oder 38B) befindet, dadurch gekennzeichnet, daß (a) ein Loch (42) in dem Polster zwischen den Bandabschnitten vorgesehen ist, durch das der Katheter (36) in eine Richtung quer zu dem Polster (32) geführt werden kann, und (b) eine Oberfläche (38A oder 38B) eines Bandabschnitts mehrfach verwendbares Haftmittel trägt und zu dem anderen Bandabschnitt (38B oder 38A) weist, so daß die zueinander weisenden Oberflächen der Bandabschnitte zeitweise auf jeder Seite des Katheters aneinandergeklebt werden können, um dadurch den Katheter zu ummanteln und ihn in einer solchen Position zu halten, daß er quer zu dem Polster verläuft, wobei die entfernten Enden (44A, 44B) der Bandabschnitte haftmittelfrei sind, so daß sie dazu verwendet werden können, ein Auseinanderziehen der Bandabschnitte zu erleichtern, und das Haftmittel außerdem so angeordnet ist, daß es einen haftenden Eingriff mit dem Katheter herstellt, um dessen Längsbewegung gegenüber dem Polster (32) zu verhindern.

2. Katheterhalterung nach Anspruch 1, wobei das Polster von seiner Kante nach innen mit einem Schlitz (46) versehen ist und der Schlitz in das Loch mündet.

3. Katheterhalterung nach Anspruch 1 oder 2, wobei das medizinische Haftmaterial (32) ein Kunststoffhaftmaterial mit einem Gemisch aus einem wasserlöslichen oder wasserquellbaren Hydrokolloid und einem wasserunlöslichen viskosen elastischen Bindemittel ist.

4. Katheterhalterung nach einem der vorausgegangenen Ansprüche, wobei die Breite jedes Bandabschnitts (38A, 38B) 4 bis 6,5d beträgt, worin d der Durchmesser des Lochs (42) ist.

5. Katheterhalterung nach Anspruch 4, wobei die Breite der Bandabschnitte (38A, 38B) 5d bis 6d beträgt.

6. Katheterhalterung nach einem der vorausgegangenen Ansprüche in Kombination mit einem Katheter und einem Beutel zum Aufnehmen von Galle, die in den Beutel über den Katheter geleitet wird, wobei der Beutel so gestaltet ist, daß er an dem Träger durch das medzinische Haftmittelpolster (32) gehalten wird.

## Revendications

1. Porte-cathéter qui comprend un tampon (32) de matière adhésive de qualité médicale dont une surface est destinée à être fixée à la peau d'un porteur, un moyen en forme de bande ou de ruban fixé à l'autre surface du tampon et présentant deux parties (38A,38B) qui partent de ladite autre surface et se trouvent en regard l'une de l'autre, de l'adhésif étant prévu sur une surface d'au moins une partie de bande (38A ou 38B), caractérisé en ce que (a) il est prévu un trou (42) dans le tampon entre les parties de bande, par lequel on peut faire passer le cathéter (36) dans une direction transversale au tampon (32), et (b) une surface (38A ou 38B) d'une partie de bande porte un adhésif multi-usages et est tournée vers l'autre partie de bande (38B ou 38A) de telle sorte que les surfaces en regard des parties de bande peuvent être temporairement collées l'une à l'autre de chaque côté du cathéter de façon à enfermer le cathéter et à le maintenir dans une position telle qu'il est dirigé transversalement au tampon, les extrémités distales (44A,44B) des parties de bande étant dépourvues dudit adhésif de façon à pouvoir être utilisées pour faciliter le décollement mutuel des parties de bande, et ledit adhésif étant également disposé de façon à venir coller le cathéter et empêcher son déplacement longitudinal par rapport au tampon (32).

2. Porte-cathéter selon la revendication 1, dans lequel le tampon est fendu (46) vers l'intérieur à partir de son bord, la fente rejoignant le trou.

3. Porte-cathéter selon la revendication 1 ou 2, dans lequel la matière adhésive de qualité médicale (32) est une matière adhésive plastique comprenant un mélange d'un hydrocolloïde soluble dans l'eau ou gonflant dans l'eau et d'un liant élastique visqueux insoluble dans l'eau.

4. Porte-cathéter selon l'une quelconque des revendications précédentes, dans lequel la largeur de l'une ou l'autre des parties de bande (38A,38B) est de 4 à 6,5 d, d étant le diamètre du trou (42).

5. Porte-cathéter selon la revendication 4, dans lequel la largeur des parties de bande (38A,38B) est de 5d à 6d.

6. Porte-cathéter selon l'une quelconque des revendications précédentes, combiné à un cathéter et à un sac destiné à recevoir la bile qui arrive dans le sac par le cathéter, le sac étant conçu pour être maintenu sur le porteur par le tampon (32) d'adhésif de qualité médicale.
